# EUROPÄISCHE PATENTSCHRIFT

(11) **EP 3 290 070 B1**
(45) Veröffentlichungstag und Bekanntmachung des Hinweises auf die Patenterteilung: **19.12.2018**
(21) Anmeldenummer: 16187309.6
(22) Anmeldetag: 06.09.2016
(51) Int. Cl.: A61M 3/02, A61M 5/14, A61J 1/20

(54) **BEHÄLTER ZUM FASSEN EINER FLÜSSIGKEIT**
CONTAINER FOR CONTAINING A LIQUID
RECIPIENT POUR UN LIQUIDE

(43) Veröffentlichungstag der Anmeldung: 07.03.2018
(73) Patentinhaber: Fritz Ruck Ophthalmologische Systeme GmbH, 52249 Eschweiler (DE)
(72) Erfinder: Klomp, Manfred, 6336 AM Hulsberg (NL)
(74) Vertreter: Schwarz & Partner Patentanwälte OG

(56) Entgegenhaltungen:
- WO-A1-96/37245
- US-A1- 2003 083 640
- US-A1- 2014 276 639

## Beschreibung

Die Erfindung betrifft einen Behälter zum Fassen einer Flüssigkeit, insbesondere einer Infusionsflüssigkeit oder Irrigationsflüssigkeit, mit einer die Flüssigkeit enthaltenden Flüssigkeitsphase und einer Gasphase, wobei in Betriebslage im unteren Bereich des Behälters eine Flüssigkeitsöffnung ausgebildet ist und wobei der Behälter eine Gasöffnung aufweist.

Es sind Behälter für den Einsatz im medizinischen Bereich bekannt, die eine Flüssigkeitsphase und eine Gasphase enthalten und in Betriebslage im unteren Bereich des Behälters eine Öffnung aufweisen. Die Öffnung ist für den Transport des Behälters mit einem Siegel verschlossen, welches zur Entnahme der Flüssigkeit aus dem Behälter mittels eines Dorns durchstochen werden muss. So ein Behälter wird zum Beispiel bei Infusionen verwendet, wobei die Entnahme der Flüssigkeit aus dem Behälter durch Schwerkraft erfolgt.

Solche Behälter können aber auch in Augenoperationsgeräten eingesetzt werden, wobei zur Entnahme der Flüssigkeit aus dem Behälter das Siegel des Behälters durch zwei Dorne durchstochen werden muss, wobei ein erster Dorn eine Länge aufweist, sodass dieser in Betriebslage des Behälters in die Gasphase des Behälters reicht und ein zweiter Dorn eine Länge aufweist, sodass dieser in Betriebslage des Behälters in die Flüssigkeitsphase reicht. Über den ersten Dorn wird während einer Operation mittels einer über Schläuche oder Rohre an den ersten Dorn angeschlossenen geregelten Verdichtereinheit Gas in den Behälter zugeführt, wodurch die Flüssigkeit durch den zweiten Dorn mit einem bestimmten Druck aus dem Behälter gedrückt wird. Infolge kann der Abgabedruck der Flüssigkeit aus dem Behälter unabhängig von einer Füllmenge des Behälters mit Flüssigkeit und unabhängig von einer Höhe eines Aufhängepunktes des Behälters geregelt werden.

Durch die Länge des in die Gasphase reichenden ersten Dorns ist der Nachteil erhalten, dass es für eine Person bzw. einen Operateur schwierig ist beim Wechsel des Behälters mit dem langen Dorn auf Anhieb das Siegel zu treffen und dieses zu durchstechen. Auch kann es vorkommen, dass nach dem Durchstechen des Siegels auch noch die Seitenwand des Behälters durchstochen wird, wonach die Flüssigkeit unkontrolliert ausläuft und der Behälter entsorgt werden muss. Damit steigt nicht nur das Verletzungsrisiko für das die Operation durchführende Personal, sondern auch das Risiko einer Kontamination der Flüssigkeit. Ferner ist das Risiko hoch, dass beim Durchstechen des Siegels Flüssigkeit in das längere Rohr läuft, welches in weiterer Folge der Schwerkraft folgt und in Leitungen oder Schläuchen zwischen dem Behälter und der Verdichtereinheit zum Stehen kommt oder im aller schlechtesten Fall in die Verdichtereinheit läuft, wodurch diese plötzlich ausfallen kann. Das Vorsehen von Wasserabscheidern zwischen der Verdichteinheit und dem ersten Dorn verhindert zwar das Sammeln von Wasser in den Leitungen oder Schläuchen, aber nur bis zu einem gewissen Ausmaß. Darüber hinaus sind die Wasserabscheider schlecht bis gar nicht zu reinigen und müssen daher regelmäßig getauscht werden.

Um diese Nachteile zu umgehen wurden Behälter entwickelt, welche aus einer elastischen Kunststofffolie gebildet sind und zwei Kammern aufweisen. Eine erste Kammer ist zum Fassen eines ersten Fluids, insbesondere Gas, ausgebildet und eine zweite Kammer ist zum Fassen eines zweiten Fluids, insbesondere Flüssigkeit, ausgebildet. Jede der beiden Kammern weist in Betriebslage des Behälters im unteren Bereich eine Öffnung auf, wobei durch Zufuhr des ersten Fluids in die erste Kammer das zweite Fluid aus der zweiten Kammer gedrückt wird. Das erste Fluid und das zweite Fluid sind durch die Ausbildung des Behälters immer von einander getrennt. So ein Behälter ist zum Beispiel aus der Druckschrift US 2008/0065030 A1 bekannt.

Als nachteilig bei dem Behälter bekannt aus der Druckschrift US 2008/0065030 A1 erweist sich, dass die Herstellung des Behälters sehr aufwendig und teuer ist. Ferner ist ein Druck in der Flüssigkeitsphase schlecht regelbar bzw. werden Druckänderung nur verzögert von der Gasphase in die Flüssigkeitsphase übertragen, wodurch der Abgabedruck, mit der die Flüssigkeit durch die Öffnung aus dem Behälter gedrückt wird, schlecht regelbar ist. Dies ist aber für Augenoperationsgeräte unabdingbar, denn sowohl bei zu kleinen Drücken, als auch bei zu großen Drücken kann es während der Operation zu einer irreparablen Beschädigung von Gewebe des Auges kommen. Ein vergleichbarer Behälter ist auch aus WO 96/37245 bekannt. Es ist daher die Aufgabe der vorliegenden Erfindung einen Behälter bereit zu stellen, der einfach zu handhaben ist und bei dem Druckänderungen in der Gasphase ohne Verzögerung in die Flüssigkeitsphase übertragen werden.

Erfindungsgemäß wird die Aufgabenstellung durch einen Behälter gemäß Anspruch 1 dadurch gelöst, dass die Gasöffnung durch ein Gasrohr gebildet ist, welches mit dem Behälter unlösbar verbunden ist sowie ein erstes Gasrohrende und ein zweites Gasrohrende aufweist, wobei das erste Gasrohrende in die Gasphase des Behälters reicht und mit einem Verschluss versehen ist und wobei das zweite Gasrohrende außerhalb des Behälters mündet.

Hierdurch ist der Vorteil erhalten, dass in Betriebslage des Behälters die Gasöffnung in die Gasphase reicht und die Flüssigkeitsöffnung in die Flüssigkeitsphase reicht, wodurch das Vorsehen von Dornen entsprechend dem Stand der Technik, mit denen ein Siegel beim Wechseln der Flasche durchstochen werden muss, vermieden werden kann. Ein Anschließen des Behälters an Schläuche oder Leitungen erfolgt an einer Außenseite des Behälters durch simples Anstecken der Schläuche oder Leitungen an die Gasöffnung bzw. die Flüssigkeitsöffnung, wodurch die Handhabung des Behälters wesentlich erleichtert wird. Da die Gasöffnung direkt in die Gasphase reicht und die Flüssigkeitsöffnung direkt in die Flüssigkeitsphase reicht und da die Gasphase und die Flüssigkeitsphase nicht voneinander getrennt sind, wird eine Druckänderung in der Gasphase direkt und ohne Verzögerung in die Flüssigkeitsphase übertragen.

Der am Ende des Gasrohrs angeordnete Verschluss dient dazu, dass während dem Transport des Behälters, also wenn er sich in einer Lage befindet, die nicht seiner Betriebslage entspricht, keine Flüssigkeit in das Gasrohr laufen kann. Der Verschluss wird erst bei in Betriebslage befindlichem Behälter kurz vor Abgabe der Flüssigkeit im Behälter geöffnet. Vorteilhaft ist dabei der Verschluss durch eine Brechsicherung oder einen Stopfen gebildet, wobei der Stopfen entweder, insbesondere bei elastischen Behältern, manuell entfernbar ist oder durch Druckbeaufschlagung der Gasöffnung entfernbar ist.

Vorteilhaft sind/ist die Flüssigkeitsöffnung und/oder das zweite Gasrohrende des Gasrohrs mit einer Membran verschlossen, die beim Anschließen von Leitungen an der Flüssigkeitsöffnung bzw. an der Gasöffnung jeweils entfernt bzw. durchstoßen wird. In einer weiteren Ausführungsvariante sind/ist die Flüssigkeitsöffnung und/oder das zweite Gasrohrende des Gasrohrs mit einem Stopfen aus Gummi verschlossen. Hierdurch wird eine Kontamination eines Inneren des Gasrohrs, bzw. ein Austreten von Flüssigkeit aus der Flüssigkeitsöffnung während des Transports verhindert.

Erfindungsgemäß ist der Behälter aus einer gefalteten und an den Rändern verschweißten elastischen Kunststofffolie gebildet. Bevorzugt ist die Flüssigkeitsöffnung durch ein Flüssigkeitsrohr gebildet, wobei das Flüssigkeitsrohr und das Gasrohr jeweils ein Kunststoffrohr sind. Zweckmäßig ist sowohl das Gasrohr, als auch das Flüssigkeitsrohr mit der Kunststofffolie verschweißt. Hierdurch ist der Vorteil erhalten, dass die Behälter sehr einfach mit geringem Materialaufwand in Serienfertigung zu fertigen und somit kostengünstig sind.

Weitere vorteilhafte Ausführungsvarianten des erfindungsgemäßen Behälters werden in weiterer Folge anhand der Figuren näher erläutert.
Figur 1 zeigt einer erste Ausführungsvariante eines erfindungsgemäßen Behälters in Betriebslage in einer schematischen Seitenansicht.
Figur 2 zeigt eine weitere Ausführungsvariante eines erfindungsgemäßen Behälters in Betriebslage in einer schematischen Seitenansicht.

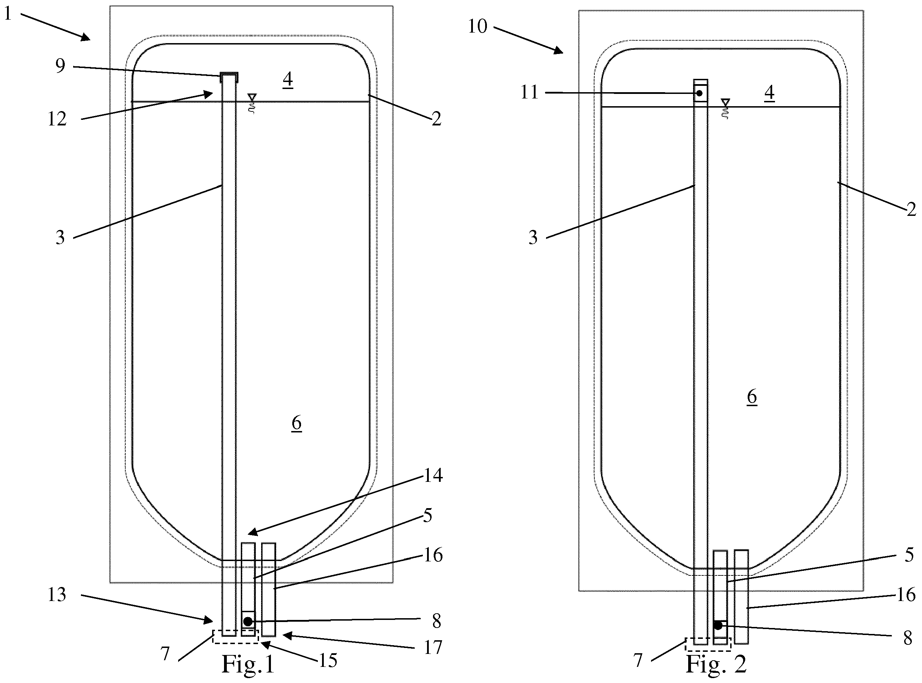

Figur 1 zeigt eine erste Ausführungsvariante des erfindungsgemäßen Behälters 1 in Betriebslage in einer schematischen Seitenansicht. Der Behälter 1 ist durch eine elastische Kunststofffolie gebildet, welche umgefaltet und an den Rändern entlang der Linie 2 verschweißt ist, und umfasst eine Flüssigkeitsphase 6 und eine Gasphase 4. Der Behälter 1 weist eine Gasöffnung auf, welche durch ein Gasrohr 3 gebildet ist. Das Gasrohr 3 ist mit der Kunststofffolie des Behälters 1 fest verschweißt, wobei ein erstes Gasrohrende 12 des Gasrohrs 3 in Betriebslage in die Gasphase 4 reicht und ein zweites Gasrohrende 13 des Gasrohrs 3 im unteren Bereich des Behälters 1 aus dem Behälter 1 ragt. Der Behälter 1 weist ferner eine Flüssigkeitsöffnung auf, welche durch ein Flüssigkeitsrohr 5 gebildet ist. Das Flüssigkeitsrohr 5 ist mit der Kunststofffolie des Behälters 1 fest verschweißt, wobei ein erstes Flüssigkeitsrohrende 14 des Flüssigkeitsrohrs 5 in Betriebslage in die Flüssigkeitsphase 6 reicht und ein zweites Flüssigkeitsrohrende 15 des Flüssigkeitsrohrs 5 im unteren Bereich des Behälters 1 aus dem Behälter 1 ragt. Der Behälter 1 weist darüber hinaus eine Injektionsöffnung auf, welche durch ein Injektionsrohr 16 gebildet ist. Das Injektionsrohr 16 ist mit der Kunststofffolie des Behälters 1 fest verschweißt und dient zur Zugabe von Medikamenten in die Flüssigkeitsphase 6. Ein nach außen hin mündendes Injektionsrohrende 17 des Injektionsrohrs 16 ist mit einem nicht dargestellten Gummistopfen verschlossen, wodurch ein Austreten von Flüssigkeit aus dem Behälter 1 durch das Injektionsrohr 16 vermieden wird. Eine Zugabe von Medikamenten in die Flüssigkeitsphase 6 kann zum Beispiel mittels einer Spritze erfolgen, wobei mit einer Nadel der Spritze bei der Zugabe von Medikamenten der Stopfen einfach durchstoßen wird. Das zweite Gasrohrende 13 und das zweite Flüssigkeitsrohrende 15 bilden eine Anschlusseinheit 7. Hierdurch ist der Vorteil erhalten, dass die Flüssigkeitsöffnung und die Gasöffnung mit einem Handgriff an Schläuche oder sonstige Einrichtungen anschließbar sind. Zweckmäßig weisen das zweite Gasrohrende 13 und das zweite Flüssigkeitsrohrende 15 dazu jeweils eine Luer-Verbindung auf, welche mit einer Membran verschlossen sind um während eines Transports des Behälters 1 eine Kontamination eines Inneren des Gasrohrs 3 und des Flüssigkeitsrohrs 5 zu vermeiden. Wird ein Schlauch oder eine Leitung an die Luer-Verbindung angeschlossen, wird gleichzeitig auch die Membran durchstoßen.

Im Flüssigkeitsrohr 5 ist eine zweite Brechsicherung 8 ausgebildet und am ersten Gasrohrende 12 des Gasrohrs 3 ist ein durch einen Stopfen 9 gebildeter Verschluss ausgebildet. Der Stopfen 9 ist als Kunststoffkappe ausgebildet, die durch geeignete Wahl des Innendurchmessers mittels Reibung zuverlässig auf dem ersten Gasrohrende 12 des Gasrohrs 3 sitzt.

In weiterer Folge wird die Verwendung des Behälters 1 in einem Augenoperationsgerät näher beschrieben, wobei die Flüssigkeit im Behälter 1 durch eine Irrigationsflüssigkeit gebildet ist. Das Augenoperationsgerät umfasst ferner ein chirurgisches Handstück, eine Regeleinrichtung und eine Verdichtereinheit. Das chirurgische Handstück ist mit einem Werkzeug zum Durchführen einer Augenoperation ausgestattet und mittels eines Schlauchs mit dem zweiten Flüssigkeitsrohrende 15 des zweiten Flüssigkeitsrohrs 5 verbunden. Die Verdichtereinheit ist durch eine Pumpe gebildet, ist zum Fördern von Gas ausgebildet und ist mittels eines Schlauchs an das zweite Gasrohrende 13 des Gasrohrs 3 angeschlossen. Mittels der Regeleinrichtung ist eine Abgabe von Flüssigkeit in das Auge am chirurgischen Handstück regelbar, wobei ein Druck, mit dem die Flüssigkeit aus dem chirurgischen Handstück abgegeben wird, über die Pumpe einstellbar ist. Vor der Operation wird der Behälter 1 in seine Betriebslage gemäß Figur 1 gebracht, wobei dieser vorteilhaft mittels einer nicht dargestellten Öse auf einem Hacken aufgehängt ist. Durch Aufbringen eines Drucks mittels der Verdichtereinheit wird der Stopfen 9 von seiner das erste Gasrohrende 12 des Gasrohrs 3 verschließenden Verschlussstellung in eine das Gasrohr 3 freigebende Freigabestellung gedrückt. In Freigabestellung kann der Stopfen 9 auf der Oberfläche der Flüssigkeit 6 schwimmen oder aber in einer nach oben verschobenen Stellung noch auf dem ersten Gasrohrende 12 sitzen, wobei Öffnungen in der Umfangswand des Stopfens 9 den Durchtritt von Gas von der Gasphase 4 in das Gasrohr 3 ermöglichen. Erst hierdurch ist die Gasphase des Behälters 1 direkt mit der Verdichtereinheit zum Kommunizieren verbunden. In weiterer Folge wird die Brechsicherung 8 durch Knicken dieser geöffnet und das Flüssigkeitsrohr 5 freigegeben. Die Flüssigkeit strömt zum chirurgischen Handstück und wird gesteuert durch die Regeleinrichtung an das Auge abgegeben.

In einer weiteren Ausführungsvariante reicht das erste Gasrohrende 12 des Gasrohrs 3 vom in Betriebslage oberen Bereich des Behälters 1 in die Gasphase 4 des Behälters 1.

Figur 2 zeigt eine weitere Ausführungsvariante eines erfindungsgemäßen Behälters 10 in Betriebslage in einer schematischen Seitenansicht. Der Behälter 10 unterscheidet sich zum dem in Figur 1 dargestellten Behälter 1 dadurch, dass der Behälter 10 statt eines Stopfens 9 eine erste Brechsicherung 11 aufweist, wobei erst durch Knicken dieser das Gasrohr 3 freigegeben ist.

Es kann noch erwähnt werden, dass ein erfindungsgemäßer Behälter bei einer Vielzahl an Geräten der unterschiedlichsten Fachgebiete verwendet werden kann. So ein Behälter könnte zum Beispiel zur Abgabe von Farben in Farbauftragsmaschinen, oder zur Ausgabe von Harzen oder Klebern in einer Auftragsmaschine zum Verkleben von Materialien ausgebildet sein.

## Patentansprüche

1. Behälter (1, 10) zum Fassen einer Flüssigkeit, insbesondere einer Infusionsflüssigkeit oder Irrigationsflüssigkeit, mit einer die Flüssigkeit enthaltenden Flüssigkeitsphase (6) und einer Gasphase (4), wobei in Betriebslage im unteren Bereich des Behälters (1, 10) eine Flüssigkeitsöffnung ausgebildet ist und wobei der Behälter eine Gasöffnung aufweist" welche Gasöffnung durch ein Gasrohr (3) gebildet ist, das ein erstes Gasrohrende (12) und ein zweites Gasrohrende (13) aufweist, wobei das erste Gasrohrende (12) in die Gasphase (4) des Behälters (1, 10) reicht und wobei das zweite Gasrohrende (13) außerhalb des Behälters (1, 10) mündet, **dadurch gekennzeichnet,**
**dass** das Gasrohr (3) unlösbar mit dem Behälter (1, 10) verbunden ist,
**dass** das erste Gasrohrende (12) mit einem Verschluss versehen ist,
**dass** das Gasrohr (3) mit dem ersten Gasrohrende (12) von einem in Betriebslage unteren Bereich des Behälters (1, 10) oder von einem in Betriebslage oberen Bereich des Behälters (1, 10) in die Gasphase (4) reicht und
**dass** der Behälter (1, 10) aus einer gefalteten und an den Rändern verschweißten Kunststofffolie gebildet ist, wobei das Gasrohr (3) mit der Kunststofffolie verschweißt ist.

2. Behälter (1, 10) nach Anspruch 1, **dadurch gekennzeichnet, dass** der Verschluss durch eine erste Brechsicherung (11) gebildet ist, wobei die Gasöffnung erst durch Knicken der ersten Brechsicherung (11) frei gegeben ist.

3. Behälter (1, 10) nach Anspruch 1, **dadurch gekennzeichnet, dass** der Verschluss durch einen Stopfen (9) gebildet ist, welcher von einer die Gasöffnung verschließenden Verschlussstellung in eine die Gasöffnung öffnende Freigabestellung verlagerbar ist.

4. Behälter (1, 10) nach einem der Ansprüche 1 bis 3, **dadurch gekennzeichnet, dass** der Behälter (1, 10) elastisch ausgebildet ist und insbesondere aus Kunststoff besteht.

5. Behälter (1, 10) nach einem der Ansprüche 1 bis 4, **dadurch gekennzeichnet, dass** die Flüssigkeitsöffnung durch ein Flüssigkeitsrohr (5) gebildet ist, welches mit der Kunststofffolie verschweißt ist sowie ein erstes Flüssigkeitsrohrende (14) und ein zweites Flüssigkeitsrohrende (15) aufweist, wobei das erstes Flüssigkeitsrohrende (14) in die Flüssigkeitsphase (6) reicht und das zweite Flüssigkeitsrohrende (15) außerhalb des Behälters (1, 10) mündet.

6. Behälter (1, 10) nach Anspruch 5, **dadurch gekennzeichnet, dass** bei dem Gasrohr (3), das mit dem ersten Gasrohrende (12) vom in Betriebslage unteren Bereich des Behälters (1, 10) in die Gasphase (4) reicht, das zweite Gasrohrende (13) und das zweite Flüssigkeitsrohrende (15) eine Anschlusseinheit (7) bilden.

7. Behälter (1, 10) nach einem der Ansprüche 5 oder 6, **dadurch gekennzeichnet, dass** das zweite Gasrohrende (13) und/ oder das zweite Flüssigkeitsrohrende (15) eine Luer-Verbindung aufweist/en.

8. Behälter (1, 10) nach einem der Ansprüche 1 bis 7, **dadurch gekennzeichnet, dass** die Flüssigkeitsöffnung eine zweite Brechsicherung (8) aufweist, wobei die Flüssigkeitsöffnung erst durch Knicken der zweiten Brechsicherung (8) frei gegeben ist.

9. Augenoperationsgerät umfassend ein chirurgisches Handstück, eine Regeleinrichtung und eine Verdichtereinheit, **dadurch gekennzeichnet, dass** das Augenoperationsgerät einen Behälter (1, 10) nach einem der Ansprüche 1 bis 8 aufweist, wobei das chirurgische Handstück mittels eines Schlauchs an die Flüssigkeitsöffnung angeschlossen ist, wobei die Verdichtereinheit an die Gasöffnung angeschlossen ist und zur Regelung einer Gaszufuhr in den Behälter (1, 10) ausgebildet ist und wobei durch die Regeleinrichtung eine Abgabe von Flüssigkeit am chirurgischen Handstück regelbar ist.

## Claims

1. A container (1, 10) for receiving a fluid, in particular an infusion fluid or irrigation fluid, comprising a fluid phase (6) containing the fluid and a gas phase (4), wherein a fluid port is formed in the operating position in the lower region of the container (1, 10) and wherein the container has a gas port, which gas port is formed by a gas pipe (3) comprising a first gas pipe end (12) and a second gas pipe end (13), wherein the first gas pipe end (12) extends into the gas phase (4) of the container (1, 10) and the second gas pipe end (13) ends outside of the container (1, 10), **characterized in that**
the gas pipe (3) is inseparably connected to the container (1, 10),
the first gas pipe end (12) is provided with a closure,
the gas pipe (3) extends with the first gas pipe end (12) from a region of the container (1, 10) which is lower in the operating position or from a region of the container (1, 10) which is upper in the operating position into the gas phase (4), and
the container (1, 10) is formed from a folded plastic film welded at the edges, with the gas pipe (3) being welded to the plastic film.

2. A container (1, 10) according to claim 1, **characterized in that** the closure is formed by a first anti-breakage device (11), wherein the gas port is released only by buckling the first anti-breakage device (11).

3. A container (1, 10) according to claim 1, **characterized in that** the closure is formed by a plug (9) which can be shifted from a closed position closing the gas port into a release position opening the gas port.

4. A container (1, 10) according to any of claims 1 to 3, **characterized in that** the container (1, 10) is flexible and is made in particular of plastic.

5. A container (1, 10) according to any of claims 1 to 4, **characterized in that** the fluid port is formed by a fluid pipe (5) which is welded to the plastic film and has a first fluid pipe end (14) and a second fluid pipe end (15), wherein the first fluid pipe end (14) extends into the fluid phase (6) and the second fluid pipe end (15) ends outside of the container (1, 10).

6. A container (1, 10) according to claim 5, **characterized in that**, in the case of the gas pipe (3) extending with the first gas pipe end (12) from a region of the container (1, 10) which is lower in the operating position into the gas phase (4), the second gas pipe end (13) and the second fluid pipe end (15) form a connection unit (7).

7. A container (1, 10) according to any of claims 5 or 6, **characterized in that** the second gas pipe end (13) and/or the second fluid pipe end (15) comprise(s) a Luer connection.

8. A container (1, 10) according to any of claims 1 to 7, **characterized in that** the fluid port exhibits a second anti-breakage device (8), wherein the fluid port is released only by buckling the second anti-breakage device (8).

9. An ophthalmic surgical device comprising a surgical handpiece, a control device and a compressor unit, **characterized in that** the ophthalmic surgical device comprises a container (1, 10) according to any of claims 1 to 8, wherein the surgical handpiece is connected to the fluid port by means of a tube, wherein the compressor unit is connected to the gas port and is configured for controlling a gas supply into the container (1, 10) and wherein a delivery of fluid at the surgical handpiece is adjustable by means of the control device.

## Revendications

1. Récipient (1, 10) pour contenir un liquide, en particulier un liquide de perfusion ou un liquide d'irrigation, avec une phase liquide (6) qui contient le liquide et avec une phase gazeuse (4), dans lequel dans la position de service il se forme dans la zone inférieure du récipient (1, 10) une ouverture à liquide et le récipient comporte une ouverture à gaz, celle-ci étant formée par un tube à gaz (3) qui présente une première extrémité (12) et une deuxième extrémité (13), dans lequel la première extrémité (12) du tube à gaz s'étend jusque dans la phase gazeuse (4) du récipient (1, 10) et la deuxième extrémité (13) du tube à gaz débouche à l'extérieur du récipient (1, 10), **caractérisé en ce que**
le tube à gaz (3) est relié avec le récipient (1, 10) sans pouvoir être détaché, la première extrémité (12) du tube à gaz est dotée d'un obturateur,
le tube à gaz (3) s'étend avec la première extrémité (12) jusque dans la phase gazeuse (4) soit depuis une zone inférieure, dans la position de service, du récipient (1, 10), soit depuis une zone supérieure, dans la position de service, du récipient (1, 10), et
le récipient (1, 10) est constitué à partir d'une feuille de matière plastique pliée et soudée au niveau des bordures, et le tube à gaz (3) est soudé avec la feuille de matière plastique.

2. Récipient (1, 10) selon la revendication 1, **caractérisé en ce que** l'obturateur est formé par une première fermeture frangible (11), et l'ouverture à gaz est libérée uniquement par pliage de la première fermeture frangible (11).

3. Récipient (1, 10) selon la revendication 1, **caractérisé en ce que** l'obturateur est formé par un bouchon (9) qui est déplaçable depuis une position d'obturation qui obture l'ouverture à gaz jusque dans une position de libération qui ouvre l'ouverture à gaz.

4. Récipient (1, 10) selon l'une des revendications 1 à 3, **caractérisé en ce que** le récipient (1, 10) est réalisé de manière élastique et est en particulier constitué en matière plastique.

5. Récipient (1, 10) selon l'une des revendications 1 à 4, **caractérisé en ce que** l'ouverture à liquide est formée par un tube à liquide (5) qui est soudé avec la feuille de matière plastique et qui présente une première extrémité (14) et une deuxième extrémité (15), dans lequel la première extrémité (14) du tube à liquide s'étend jusque dans la phase liquide (6) et la deuxième extrémité (15) du tube à liquide débouche à l'extérieur du récipient (1, 10).

6. Récipient (1, 10) selon la revendication 5, **caractérisé en ce que** pour ce qui concerne le tube à gaz (3), la première extrémité (12) du tube à gaz s'étend depuis la zone inférieure, en position de service, du récipient (1, 10) jusque dans la phase gazeuse (4), et **en ce que** la deuxième extrémité (13) du tube à gaz et la deuxième extrémité (15) du tube à liquide forment une unité de raccordement (7).

7. Récipient (1, 10) selon l'une des revendications 5 ou 6, **caractérisé en ce que** la deuxième extrémité (13) du tube à gaz et/ou la deuxième extrémité (15) du tube à liquide comprend/comprennent une liaison du type Luer.

8. Récipient (1, 10) selon l'une des revendications 1 à 7, **caractérisé en ce que** l'ouverture à liquide comprend une deuxième fermeture frangible (8), et l'ouverture à liquide est libérée uniquement par pliage de la deuxième fermeture frangible (8).

9. Appareil d'opération oculaire incluant une pièce à main chirurgicale, un dispositif de régulation et une unité à compresseur, **caractérisé en ce que** l'appareil d'opération oculaire comprend un récipient (1, 10) selon l'une des revendications 1 à 8, et la pièce à main chirurgicale est raccordée au moyen d'un tuyau souple à l'ouverture à liquide, l'unité à compresseur est raccordée à l'ouverture à gaz et est réalisée pour la régulation d'une amenée de gaz dans le récipient (1, 10), et une distribution de liquide à la pièce à main chirurgicale est susceptible d'être régulée par le dispositif de régulation.
